# EUROPEAN PATENT APPLICATION

(11) **EP 1 156 334 A2**
(43) Date of publication of application: **21.11.2001**
(21) Application number: 01304178.5
(22) Date of filing: 09.05.2001
(51) Int. Cl.: G01N 33/68

(54) **Method for screening a substance having promoting activity on hair growth**

(30) Priority: 10.05.2000 JP 2000137052
(71) Applicant: Sumitomo Electric Industries, Ltd., Osaka-shi, Osaka 541-0041 (JP)
(72) Inventor: Hirai, Yohei, Sumitomo Electric Industries,Ltd., Yokohama-shi, Kanagawa 244-0844 (JP); Takebe, Kyoko, Sumitomo Electric Industries,Ltd., Yokohama-shi, Kanagawa 244-0844 (JP)
(74) Representative: Baldock, Sharon Claire

(57) **Abstract**

A method for screening a substance having promoting activity on hair growth, which comprises the steps of bringing hair follicles in telogen into contact with a test substance, and judging that the substance tested is positive when the hair follicles in telogen are led to in anagen by the substance tested, wherein the judgment is carried out by detecting expression of Hacl-1 in the hair follicles.

## Description

### Field of the Invention

The present invention relates to a method for screening a substance having promoting activity on hair growth.

### Related Art

For development of trichogenous agents and hair care products, a method is required which enables precise and simple judgment of promoting activity of a substance tested on hair growth. A method has been known so far which comprises the step of determining hair growth, based on DNA synthesis and the like as a criterion, by using a skin cultured in the presence of a test substance. However, this method has a problem in that the culture of cells is practically difficult, which results in low reproducibility of the method. Another method is available in which experimental animals are used and their skin area in anagen and hair growth are examined for respective animals, however, the method is very troublesome. Accordingly, development of a method has been desired that enables precise and simple screening of a substance having promoting activity on hair growth.

A protein expressed in differentiation from hair matrix into hair is known (Hacl-1: Huh, N. et al., The Journal of Investigative Dermatology, 102(5), pp. 716-720, 1994). The aforementioned publication suggests that a mRNA level of the protein is correlated with an activated state of follicles in hair differentiation and regeneration processes, and Fig. 5A of the publication discloses that the protein is expressed only in a certain phase. However, the publication neither suggests nor teaches as to when the protein is expressed, i.e., which of phases of anagen or telogen of hair follicles.

### Disclosure of the Invention

An object of the present invention is to provide a method which enables precise and simple screening of a test substance having promoting activity on hair growth. Another object of the present invention is to provide a method which enables precise judgment as to the growth phase of hair follicles, i.e., they are in which phases of anagen or telogen.

The inventors of the present invention conducted intensive studies to achieve the foregoing objects. As a result, they found that Hacl-1 was expressed only specifically in anagen of hair follicles, and that precise judgment as to growth phases of hair follicles, i.e., which of anagen or telogen, was achievable by determining Hacl-1 using an antibody against Hacl-1. They also found that promoting activity of a test substance on hair growth was precisely judged by observing whether or not hair follicles are led from telogen to anagen in the presence of the substance tested. The present invention was achieved on the basis of these findings.

The present invention thus provides a method for judging whether or not hair follicles are in anagen, which comprises the step of determining presence or absence of expression of Hacl-1 in the hair follicles. According to a preferred embodiment of the aforementioned method, the step of determining the expression of Hacl-1 in the hair follicles can be carried out by using an antibody against Hacl-1.

The aforementioned judging method can be used for screening a substance having promoting activity on hair growth. The present invention thus provides a method for screening a substance having promoting activity on hair growth, which comprises the steps of bringing hair follicles in telogen into contact with a test substance, and judging that the substance tested is positive when the hair follicles in telogen are led to in anagen by the substance tested, wherein the judgment is carried out by detecting expression of Hacl-1 in the hair follicles. In this method, the expression of Hacl-1 in the hair follicles can be detected preferably by using an antibody against Hacl-1. The present invention further provides a substance having promoting activity on hair growth, which is screened by the aforementioned screening method.

According to the method of the present invention, a substance having promoting activity on hair growth can be screened extremely simply and precisely, and development of active ingredients of medicaments such as hair growth promoters can efficiently be made.

### Brief Explanation of the Drawings

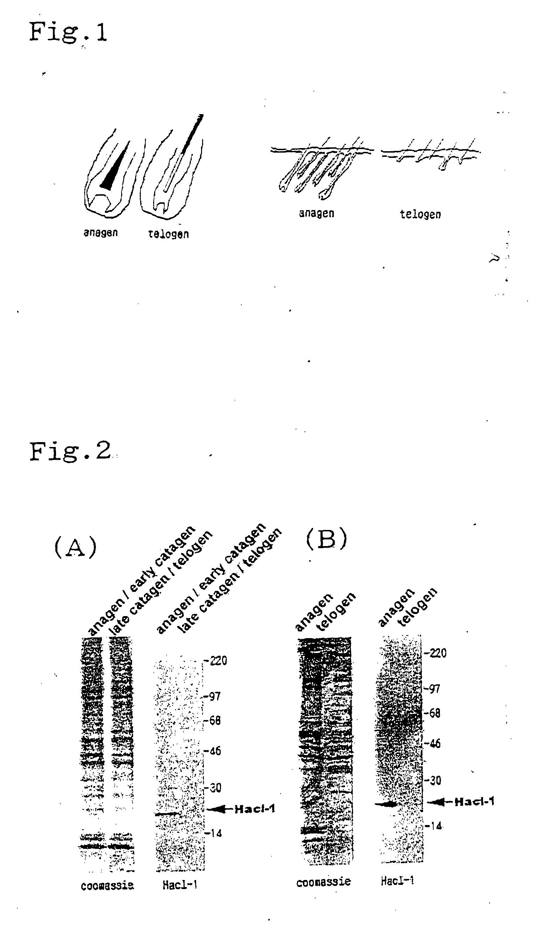

Fig. 1 depicts the criterion for judging the distinction between telogen and anagen of hair follicles by observing the tissue section under a microscope. In the figure, anagen represents a growth phase, and telogen represents a rest phase, and (A) illustrates the mouse beard and (B) illustrates the mouse skin on the back.

Fig. 2 depicts the result of the supernatant of hair follicles analyzed by SDS-polyacrylamide gel electrophoresis. (A) shows the result of the mouse beard, and (B) shows the result of the skin on the back. In the figure, "coomassie" shows the result of staining the gel after the electrophoresis with coomassie brilliant blue, and "Hacl-1" shows the result of the Western Blotting of the gel after the electrophoresis by using the antiserum against Hacl-1. Anagen represents a growth phase, catagen represents a regression phase, telogen represents a rest phase, and the arrow of Hacl-1 indicates the position of Hacl-1.

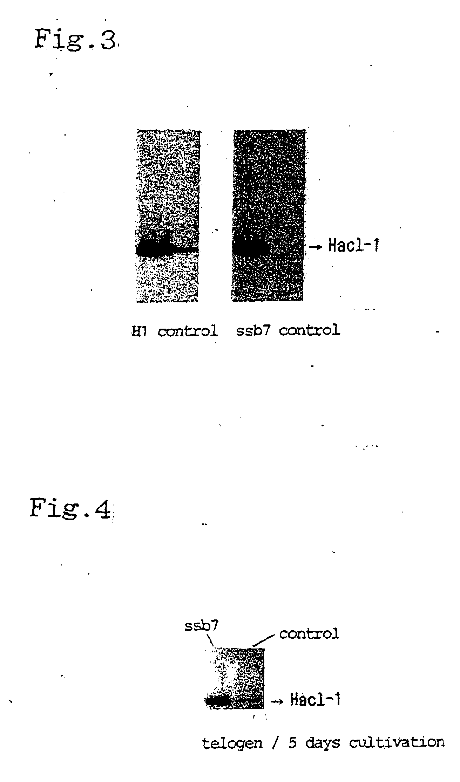

Fig. 3 depicts the results of induction of expression of Hacl-1 in hair follicles of the mouse skin and induction of anagen of the hair follicles in the presence of polypeptide H1 consisting of 104 amino acids represented by the amino acid sequence (II) described in International Publication WO 98/22506 or in the presence of oligopeptide ssb7.

Fig. 4 depicts the results of induction of expression of Hacl-1 by cultivation of the mouse beard in the presence of oligopeptide ssb7, and induction of anagen of the hair follicles.

### Best Mode for Carrying Out the Invention

The method of the present invention is for judgment as to whether or not hair follicles are in anagen, and characterized to comprise the step of detecting expression of Hacl-1 in the hair follicles. According to the findings by the inventors of the present invention, Hacl-1 is expressed only in anagen in hair follicles, and the expression is not observed in telogen. Accordingly, by detecting the expression of Hacl-1 in hair follicles, it can be judged very precisely and simply that the hair follicles are in which of the phases, telogen or anagen. Hair follicles in which the expression of Hacl-1 is observed are judged to be in anagen, whilst hair follicles in which the expression of Hacl-1 is not observed are judged to be in telogen. In the hair cycle, a short period of time after the end of anagen and before the start of telogen may sometimes be referred to as catagen. In the specification, the term "anagen" may be used to encompass early catagen, and the term "telogen" may be used to encompass late catagen.

As for Hacl-1, detailed and specific disclosures are given in Huh, N. et al., The Journal of Investigative Dermatology, 102(5), pp. 716-720, 1994, and persons skilled in the art can easily detect the protein. For example, by employing a technique used in the field of gene engineering, Hacl-1 can be expressed in a microorganism as a recombinant protein by using a gene encoding Hacl-1 which is disclosed in the aforementioned publication, and by immunizing an animal such as a rat with the resulting recombinant protein in a conventional manner, an antiserum can be obtained. By using the antiserum thus obtained, the expression of Hacl-1 can be detected in a well-known manner.

In addition, a monoclonal antibody can be prepared in a conventional manner by obtaining immunocytes from an immunized animal. By labeling the monoclonal antibody with a fluorescent dye, biotin or the like, Hacl-1 can be detected with extremely high precision in a convenient way. Techniques for preparing and labeling monoclonal antibodies are well-known in persons skilled in the art. For the detection of Hacl-1, chimera antibodies may also be used. However, it should be understood that methods for detecting the expression of Hacl-1 are not limited to those using antibodies, and persons skilled in the art can appropriately choose means for detection. The hair follicles may be derived from a human, or those derived from an animal other than a human. For the detection of the expression of Hacl-1, cells from scalp or skin, whiskers and the like can be used.

The method for screening a substance having promoting activity on hair growth provided by the present invention is characterized to comprise the steps of bringing hair follicles in telogen into contact with a test substance, and judging that the substance tested is positive when the hair follicles in telogen are led to be in anagen by the substance tested, wherein the judgment is carried out by detecting expression of Hacl-1 in the hair follicles. In general, judgment as to a growth phase of hair follicles to be brought into contact with the test substance, i.e., which of telogen or anagen, can be conducted by observing a tissue section under a microscope. Alternatively, the phase of hair follicles, telogen or anagen, may also be judged on the basis of days from birth, because, in a C57BL mouse, for example, relationship between daily age from birth and the hair cycle has been investigated in detail.

The hair follicles may be brought into contact with a test substance in any manner. An example of the method includes application or injection of a solution containing a dissolved test substance to skin involving hair follicles and the like. However, applicable methods are not limited to the above example. The solution containing a dissolved test substance may be prepared as an aqueous solution, or alternatively, may be prepared as a solution containing water and a water miscible organic solvent such as glycerin and ethanol. A test substance may be brought into contact with hair follicles through blood flow by oral or parenteral administration of the test substance to a human or an animal. Examples of the parenteral administration include administration by intravenous, intramuscular or subcutaneous injections, intravenous drip infusions, local administration by topical preparations such as creams and ointments, systemic administration by preparations for transdermal or transmucosal absorption, rectal administration by suppositories and the like. However, routes of administration are not limited to these examples.

According to the screening method of the present invention, Hacl-1 can be detected by the aforementioned method. When hair follicles in telogen are led to in anagen (i.e., the expression of Hacl-1 is detected) by bringing the hair follicles in telogen into contact with a test substance, it can be judged that the substance tested has promoting activity on hair growth. In general, for a control group prepared as a reference, a period until when hair follicles in telogen start expressing Hacl-1 is measured. Then measurement under the same conditions is carried out in the presence of a test substance. When the period until the hair follicles in telogen start expressing Hacl-1 is substantially reduced, it can be judged that the substance tested has promoting activity on hair growth. However, the judging methods are not limited to the aforementioned method.

### Examples

The present invention will be explained more specifically by referring to the following examples. However, the scope of the present invention is not limited to the following examples.

### Example 1

A DNA containing a gene encoding Hacl-1 (Huh, N. et al., The Journal of Investigative Dermatology, 102(5), pp. 716-720, 1994) was amplified by the PCR method, and by using the amplified product, Hacl-1 was expressed as a recombinant protein in Escherichia coli in a conventional manner. A rat was immunized with the recombinant Hacl-1 in a conventional manner to prepare an antiserum against Hacl-1.

Preparation of a antiserum was carried out as follows. The PCR method was carried out by using the cDNA library prepared from the beard tissue of a C3H mouse as the template to isolate a cDNA containing the entire reading frame of Hacl-1 (Huh, N. et al., J. Invest. Dermatol., 102, pp. 716-720, 1994). By using the isolated Hacl-1 gene as a template, a Hacl-1 gene added with 6 histidines at the N-terminal was isolated. The Hacl-1 gene added with 6 histidines at the N-terminal was integrated into an Escherichia coli expression vector, Pet3c (Novogen), and the vector was introduced into E. coli BL-21. The Escherichia coli was sufficiently proliferated at 22°C, and then treated by addition of IPTG at the final concentration of 1 mM to express Hacl-1 protein.

The E. coli was homogenized, and then treated with DNase and washed three times with a 2 M urea solution (pH 8.0). The resulting fraction was dissolved in 8 M urea (pH 8.0) and subjected to affinity absorption on N₁⁺-agarose gel (Quagen), and then Hacl-1 was eluted with 8 M urea (pH 3.0). The purified Hacl-1/8 M urea solution (pH 3.0) was dialyzed against PBS. Wistar rats (male, 8 weeks old) were immunized with the Hacl-1 protein which was sufficiently mixed with the adjuvant (once a month, 3 times). Blood was collected from the heart of each immunized rat, and the IgG fraction was recovered by a protein G column to obtain an antiserum.

A C57BL mouse (14 weeks old) was shaved on the back with clippers to confirm that the mouse had both skin areas in anagen and telogen as to the hair cycle based on colors of the skin. Each 200 mg of the skin areas in anagen and telogen was collected from the mouse and homogenized on ice for 2 minutes, and then suspended in a buffer (PBS). The supernatant was collected and subjected to SDS-polyacrylamide gel electrophoresis (SDS-PAGE). The respective hair follicles in telogen and anagen were distinguished by observing the tissue section under a microscope, and the criterion shown in Fig. 1 was applied for judgment. Fig. 2 shows the result of SDS-PAGE. (A) shows the result obtained from the mouse beard, and (B) shows the result obtained from the skin on the back. In the figure, "coomassie" shows the result obtained by staining the gel after the electrophoresis with coomassie brilliant blue, and "Hacl-1" shows the result of the Western Blotting of the gel after the electrophoresis by using the antiserum against Hacl-1 according to the method described in Eur. J. Biochem., 225, pp. 1133-1139, 1994.

From the densities of the bands stained with coomassie brilliant blue, it was found that the hair follicles in telogen and anagen contained almost the same amount of the protein. Western Blotting was carried out using the antiserum against Hacl-1. As a result, a clear band of Hacl-1 was observed in the sample obtained from the hair follicle in anagen, which verified the expression of Hacl-1. Whilst no band of Hacl-1 was detected in the hair follicles in telogen, which revealed that Hacl-1 was not expressed in the hair follicles in telogen.

### Example 2

Polypeptide H1 having morphogenesis promoting activity on epithelial cells, which is described in International Publication WO 98/22506 (a polypeptide consisting of 104 amino acids represented by amino acid sequence (II) disclosed in the aforementioned international publication) was expressed in Escherichia coli according to the method described in International Publication WO 98/22505. As a control, a supernatant obtained by lysis of the Escherichia coli was used which was not transformed with the recombinant vector encoding the aforementioned polypeptide.

Separately, oligopeptides having the amino acid sequence (I): Ser-Ile-Glu-Gln-Ser-Cys-Asp-Gln-Asp-Glu were synthesized by the solid phase method using Fmoc (referred to as "pep7" in the examples). Each oligopeptide synthesized was purified by high-performance liquid chromatography (HPLC), and their purities were found to be 90% or higher by HPLC and Mass. The conditions and retention time of HPLC were as follows:
Column: ODS-UG3 (Monomeric ODS, Nomura Kagaku), 1.0 mm in inside diameter, 100 mm in length
Measurement: room temperature (25°C)
Detection: UV 214 nm, 280 nm
Eluent: gradient of solvent A and solvent B (solvent A: 0.1% trifluoroacetic acid;
solvent B: 90% acetonitrile/0.1% trifluoroacetic acid, linear concentration gradient from 5 minutes after (solvent B: 0%) to 55 minutes (solvent B: 55%)
Flow rate: 75 ml/ml
Retention time: 21.52 minutes (dimer), 20.59 minutes (monomer)

The oligopeptide was crosslinked as follows. To a solution (5 ml) of oligopeptide pep7 dissolved in PBS at the concentration of 1 mg/ml, BMH dissolved in dimethylsulfoxide (65 *µ*l) was gradually added with stirring to the final concentration of 33 *µ*g/ul, and the reaction was carried out at 4°C overnight. The mixture was further added with 6.6 ml of PBS and with a solution of cysteine hydrochloride dissolved in PBS at the concentration of 5 mg/ml (5 ml) and mixed. As a result, a solution of the S-S bridged oligopeptide at the final concentration of 0.3 mg/ml was prepared (sometimes referred to as "ssb7" in the examples, which had the retention time of 33.01 minutes by HPLC under the aforementioned conditions). A control solution was prepared by carrying out the reaction by adding the reagents in the same manner but using PBS instead of the oligopeptide solution. A solution of the bridged oligopeptide ssb7 was added with the same volume of ethanol to prepare a 50% ethanol/PBS solution at the final concentration of 0.15 mg/ml.

Promoting activity on hair growth of the aforementioned polypeptide H1 and oligopeptide ssb7 was evaluated. C57BL/6 mice are known to have sustained telogen for about 50 days from the 45-day after birth to around the 95-day, and the hair cycle of the animal is easily judged based on the skin color changes, i.e., from pink in telogen to gray or black in anagen. Immediately after telogen of the hair cycle started, C57BL/6 mice were carefully shaved on the back (about 3 × 2.5 cm²) with electric clippers for animals so as not to injure the skin, and it was confirmed that the hair cycle was in telogen based on the color and thickness of the skin. The polypeptide H1 solution (0.8 mg/ml) or the oligopeptide ssb7 solution (0.8 mg/ml) prepared above was subcutaneously injected 5 times in the amount of 0.1 ml at intervals of 24 hours, and Hacl-1 in the hair follicles was measured with time according to the method of Example 1.

As a result, a clear band of Hacl-1 was observed in each samples at 20-day in the groups injected with the aforementioned polypeptides H1 and ssb7, which revealed induction of anagen. In the control group for which samplings were made at the same time, it was found that the follicles remained in telogen, and Hacl-1 was not expressed or detected at only a very slight amount when expressed. The results are shown in Fig. 3.

### Example 3

The beard on the both sides of the mice used in Example 2 was collected and cultured by the method described in Development, 105, pp. 271-277, 1989. The beard from one side was used as a control, and that from the other side was treated with the aforementioned oligopeptide ssb7 (50 µ g/ml). After cultivation for 5 days, Hacl-1 in the cultures was determined according to the method of Example 1. As a result, expression of Hacl-1 was observed in the beard treated with the oligopeptide ssb7, but only slight expression of Hacl-1 was observed in the control. The results are shown in Fig. 4.

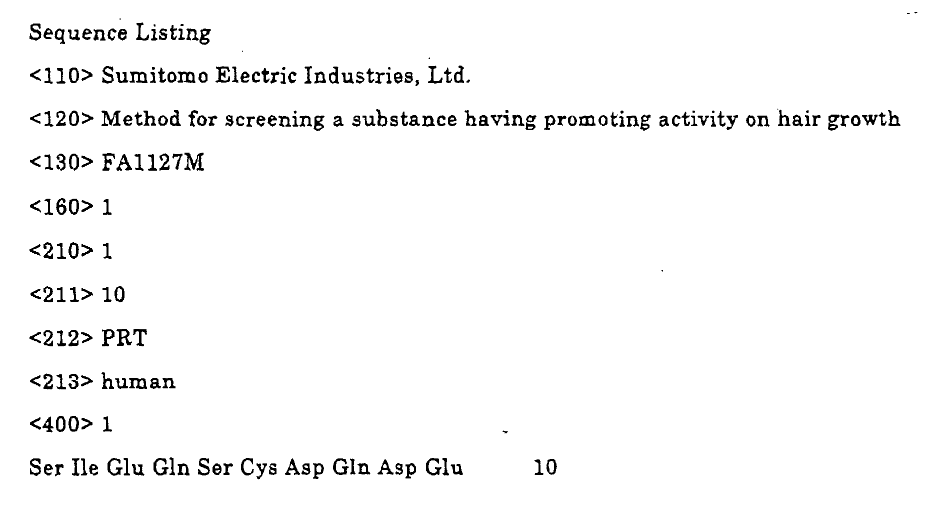

## Claims

1. A method for judging whether or not a hair follicle is in anagen or not, which comprises the step of determining presence or absence of expression of Hacl-1 in the hair follicle.

2. The method according to claim 1, wherein the step of determining expression of Hacl-1 in the hair follicle is carried out by using an antibody against Hacl-1.

3. A method for screening a substance having promoting activity on hair growth, which comprises the step of:
bringing a hair follicle in telogen into contact with a test substance, and
judging that the substance tested is positive when the hair follicle in telogen is led to in anagen by the substance tested,
and wherein the judgment is carried out by detecting expression of Hacl-1 in the hair follicle.

4. The method according to claim 3, wherein the detection of the expression of Hacl-1 in the hair follicle is carried out by using an antibody against Hacl-1.

5. A substance having promoting activity on hair growth, which is screened by the method according to claim 3 or claim 4.
